# EUROPEAN PATENT SPECIFICATION

(11) **EP 2 295 092 B1**
(45) Date of publication and mention of the grant of the patent: **14.03.2012**
(21) Application number: 10176112.0
(22) Date of filing: 10.09.2010
(51) Int. Cl.: A61M 1/34

(54) **Haemodiafiltration set**
Vorrichtung zur Hämo-Filterung und -Dialyse
Dispositif pour hémofiltration et hémodialyse

(30) Priority: 10.09.2009 IT BO20090577
(43) Date of publication of application: 16.03.2011
(73) Proprietor: Bellco S.r.l., Mirandola (IT)
(72) Inventor: Atti, Mauro, 40131, Bologna (IT); Palladino, Giuseppe, 41126, Modena (IT); Corazza, Luca, 40057, Granarolo dell' Emilia (IT)
(74) Representative: Jorio, Paolo

(56) References cited:
- EP-A1- 1 852 136
- EP-A2- 0 451 429
- WO-A1-2004/056460
- WO-A2-2008/106191
- US-A- 5 571 418

## Description

The present invention relates to a haemodiafiltration set.

Among the various dialytic techniques, the technique of haemodiafiltration is perhaps the one which has aroused the greatest interest in the clinical field. The technique of haemodiafiltration cleanses the blood by exploiting both the phenomenon of diffusion, typical of the technique of haemodialysis, and the phenomenon of convection, typical of the technique of haemofiltration.

The technique of haemodiafiltration can be performed by using one single filter, in which the blood undergoes both the diffusion phenomenon and the convection phenomenon, or via the use of two separate filters arranged in series to each other, in one of which the haemofiltration treatment is performed and in the other the haemodialysis treatment is performed.

As is known in the field, haemofiltration is performed with very porous synthetic membranes and with a blood circuit that provides a pressure gradient via the membrane.

The technique of haemofiltration involves the elimination of large quantities of liquid, which it is necessary to re-infuse into the patient's blood via the use of a re-infusion solution with appropriate composition.

In order to avoid problems relative to regulation of the electrolytic characteristics of the re-infusion solution, consequently causing possible imbalances during re-infusion, and problems of cost and storage, the Applicant has produced and patented (EP0451429B1) a haemodiafiltration set. In this haemodiafiltration set, the blood taken from the patient is passed through a haemofiltration filter and then through a haemodialysis filter, and comprises a regeneration device suitable for absorbing part of the ultrafiltrated substances to purify the ultrafiltrate and use it as a re-infusion solution.

The haemodiafiltration set described above has the drawback that it cannot remove the toxins with high molecular weight and the toxins linked to the proteins, consequently the blood is not cleansed of these substances with the disadvantages well-known to a person skilled in the art.

The object of the present invention is to produce a haemodiafiltration set, the technical characteristics of which are such as to remedy the drawbacks of the known art.

The subject of the present invention is a haemodiafiltration set, the essential characteristics of which are given in claim 1 and the preferred and/or auxiliary characteristics are given in claims 2 and 3.

For a better understanding of the invention, an embodiment is illustrated below purely by way of non-limiting example with the help of the figures of the accompanying drawing, in which:
figure 1 is a schematic view of a haemodiafiltration set subject of the present invention;
figures 2a, 2b, 3a, 3b, 4a and 4b are graphs which show the results of removal of toxins by means of the haemodiafiltration set of the present invention and by means of the known art.

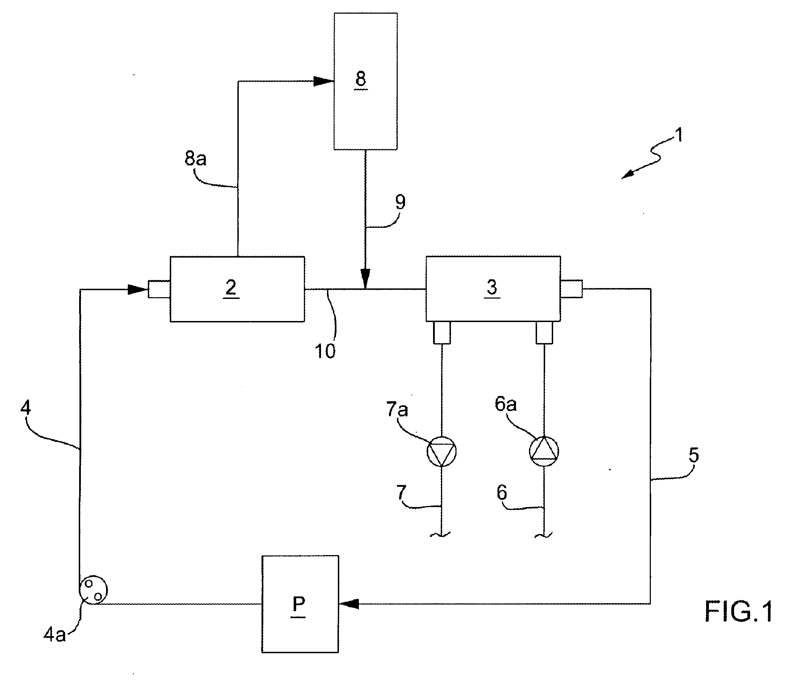

In figure 1 a haemodiafiltration set according to the present invention, with parts removed for the sake of simplicity, is indicated overall by 1.

Substantially the haemodiafiltration set 1 comprises a haemofiltration filter 2 and a haemodialysis filter 3 arranged in series to each other (known and not described in detail), an arterial line 4 for conveying the blood from a patient P to the haemofiltration filter 2, a pump 4a applied to the arterial line 4 to guarantee movement of the blood, a venous line 5 for conveying the blood from the haemodialysis filter 3 to the patient P, an inlet branch 6 and an outlet branch 7 for a dialysing solution respectively in and from the haemodialysis filter 3, a pair of pumps 6a and 7a applied respectively to the inlet branch 6 and to the outlet branch 7 of the dialysing solution, and a regeneration filter 8 for treating the ultrafiltrate from the haemofiltration filter 2 via a regeneration line 8a in order to reuse the ultrafiltrate as an infusion solution. The infusion solution is fed via an infusion line 9 into a connection line 10 for the blood arranged between the haemofiltration filter 2 and the haemodialysis filter 3.

The haemofiltration filter 2 comprises a membrane, the cut-off of which is greater than 45 KDa.

Use of the membrane with cut-off greater than 45 KDa guarantees removal of the toxins with high molecular weight and the toxins linked to the proteins. Furthermore, it has surprisingly been demonstrated that use of the membrane with cut-off greater than 45 KDa guarantees better removal also of those toxins with molecular weight such that they can be removed even with the membranes used in the machines of the known art.

Verification of removal of the toxins was performed by means of the SELDI technique and the proteomic technique.

The SELDI technique (Surface-enhanced laser desorption/ionization) is a mass spectroscopy analytical technique used for the analysis of mixtures of proteins. This technique exploits the different time of flight of ionised particles subject to a magnetic field. The SELDI technique is used for the determination of proteins in samples of tissue, blood, urine or other biological fluids.

The proteomic technique consists in the systematic identification of proteins and in their characterisation with respect to structure, function, activity, quantity and molecular interaction. The proteome is the set of all the possible proteic products expressed in a cell, including all the isoforms and the post-translational modifications. The proteome is dynamic in time, it varies in response to external factors and differs substantially between the different cell types of the same organism.

Proteomics concerns the large-scale study of proteins, in particular in relation to their structures and functions.

The verifications were performed on samples of ultrafiltrate obtained from uraemic patients undergoing dialysis by means of the haemodiafiltration set described in figure 1. In particular, the samples of ultrafiltrate were produced with a membrane having cut-off of 50KDa and used in the haemodiafiltration set subject of the present invention and, for comparison, with a membrane having a cut-off of 40KDa and used with the machines of the known art. In the figures the data relative to the membrane used in the haemodiafiltration set subject of the present invention are indicated by I, while the data relative to the comparison membrane are indicated by C. The membranes used are made of polyphenylene and marketed by the company POLIPURE COMPANY under the trade names PUREMA S and PUREMA H respectively. In particular, the membranes have a homogeneous pore structure.

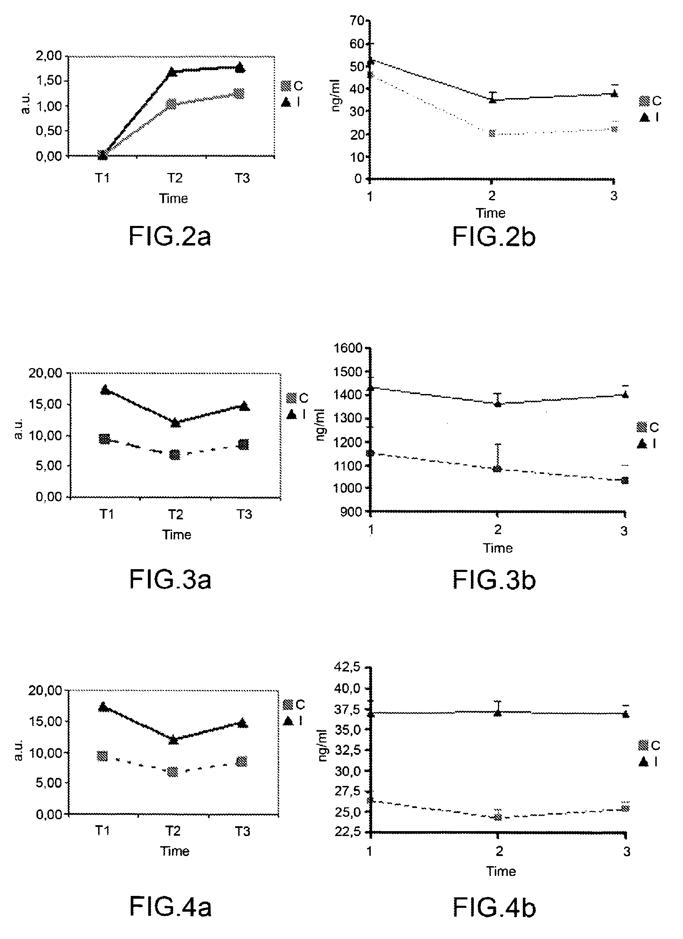

Figures 2a and 2b show the values of the concentration of α1-glycoprotein (PM=43KDa) according to time and recorded with the SELDI technique and proteomic technique respectively. From figures 2a and 2b it can be seen that in the ultrafiltrate obtained with the membrane used in the haemodiafiltration set subject of the present invention, the protein is present in higher concentrations than in the ultrafiltrate obtained from the membrane used in the machines of the known art. A higher concentration in the ultrafiltrate translates into greater removal of the protein from the blood of the patient.

Figures 3a and 3b show the values of the concentration of the complement factor D (PM=25KDa) according to time and recorded with the SELDI technique and proteomic technique respectively. From figures 3a and 3b it can be seen that, also for molecules with molecular weight below the cut-off of the membrane used in the machines of the known art, in the ultrafiltrate obtained with the membrane used in the haemodiafiltration set subject of the present invention, the protein is present in higher concentrations, consequently it is more efficiently removed from the patient's blood.

Figures 4a and 4b show the values of the concentration of Adinopectin (PM=25KDa) according to time and recorded with the SELDI technique and proteomic technique respectively. Here again, from figures 3a and 3b it can be seen that, also for molecules with molecular weight below the cut-off of the membrane used in the machines of the known art, in the ultrafiltrate obtained with the membrane used in the haemodiafiltration set subject of the present invention, the protein is present in higher concentrations, consequently it is more efficiently removed from the patient's blood.

In addition to the molecules on which the tests were performed, the haemodiafiltration set subject of the present invention is advantageously applied for removal of the substances IL-1β, IL-6, IL-8, TNF, Hyaluric Acid and λ and κ light chains.

It is important to underline that the membrane with cut-off greater than 45 KDa can be used in a haemodiafiltration set comprising a regeneration filter as described above, so as to permit recovery and re-introduction via the re-infusion solution of all those substances necessary for the blood.

## Claims

1. A haemodiafiltration set (1) comprising a haemofiltration filter (2) and a haemodialysis filter (3) arranged in series to one another, an arterial line (4) for conveying blood from a patient (P) to the haemofiltration filter (2), a venous line (5) for conveying blood from the haemodialysis filter (3) to the patient (P), an inlet branch (6) and an outlet branch (7) for a dialysing solution respectively in and from the haemodialysis filter (3), means (4a, 6a, 7a) for conveying blood and dialysing solution, a regeneration filter (8) for treating the ultrafiltrate from the haemofiltration filter (2) in order to reuse the ultrafiltrate as an infusion solution; said haemodiafiltration set (1) being **characterised in that** the haemofiltration filter (2) comprises a haemofiltration membrane made of polyphenylene and the cut-off of which is greater than 45 KDa.

2. The haemodiafiltration set (1) according to claim 1, **characterised in that** said haemofiltration membrane has a cut-off in the range between 45 and 55 KDa.

3. The haemodiafiltration set (1) according to claim 1 or 2, **characterised in that** said haemofiltration membrane has a homogenous pore structure.

## Patentansprüche

1. Eine Hämodiafiltrationsvorrichtung (1), aufweisend einen Hämofiltrationsfilter (2) und einen Hämodialysefilter (3), die hintereinander angeordnet sind, eine arterielle Leitung (4) zum Transportieren von Blut von einem Patienten (P) zum Hämofiltrationsfilter (2), eine venöse Leitung (5) zum Transportieren von Blut vom Hämodialysefilter (3) zum Patienten (P), einen Einlasszweig (6) und einen Auslasszweig (7) für eine Dialyselösung zum bzw. vom Hämodialysefilter (3), Mittel (4a, 6a, 7a) zum Transportieren von Blut und Dialyselösung, einen Regenerationsfilter (8) zum Behandeln des Ultrafiltrates vom Hämofiltrationsfilter (2), um das Ultrafiltrat als Infusionslösung wiederzuverwenden, wobei die Hämodiafiltrationsvorrichtung (1) **dadurch gekennzeichnet ist, dass** der Hämofiltrationsfilter (2) eine Hämofiltrationsmembran aufweist, die aus Polyphenylen gemacht ist und deren Trenngrenze größer als 45 KDa ist.

2. Die Hämodiafiltrationsvorrichtung (1) gemäß Anspruch 1,
**gekennzeichnet dadurch, dass** die Hämofiltrationsmembran eine Trenngrenze im Bereich zwischen 45 und 55 KDa hat.

3. Die Hämodiafiltrationsvorrichtung (1) gemäß Anspruch 1 oder 2, **gekennzeichnet dadurch, dass** die Hämofiltrationsmembran eine homogene Porenstruktur hat.

## Revendications

1. Ensemble d'hémodiafiltration (1) comprenant un filtre d'hémofiltration (2) et un filtre d'hémodialyse (3) agencés en série l'un par rapport à l'autre, une ligne artérielle (4) permettant de transporter le sang d'un patient (P) jusqu'au filtre d'hémofiltration (2), un tube veineux (5) permettant de transporter le sang du filtre d'hémodialyse (3) jusqu'au patient (P), une branche d'entrée (6) et une branche de sortie (7) pour une solution de dialyse rentrant et sortant respectivement du filtre d'hémodialyse (3), un moyen (4a, 6a, 7a) permettant de transporter le sang et la solution de dialyse, un filtre de régénération (8) permettant de traiter l'ultrafiltrat provenant du filtre d'hémofiltration (2) afin de pouvoir réutiliser l'ultrafiltrat comme une solution de perfusion ; ledit ensemble d'hémodiafiltration (1) étant **caractérisé en ce que** le filtre d'hémofiltration (2) comprend une membrane d'hémofiltration réalisée en polyphénylène et dont le seuil de coupure est supérieur à 45 kDa.

2. Ensemble d'hémodiafiltration (1) selon la revendication 1, **caractérisé en ce que** ladite membrane d'hémofiltration présente un seuil de coupure dans la plage comprise entre 45 et 55 kDa.

3. Ensemble d'hémodiafiltration (1) selon les revendications 1 ou 2, **caractérisé en ce que** ladite membrane d'hémofiltration a une structure de pores homogène.
